# EUROPEAN PATENT SPECIFICATION

(11) **EP 3 973 078 B1**
(45) Date of publication and mention of the grant of the patent: **10.07.2024**
(21) Application number: 20709308.9
(22) Date of filing: 05.03.2020
(51) Int. Cl.: C12Q 1/6883, C12Q 1/689

(54) **METHODS RELATING TO TUBERCULOSIS**
VERFAHREN IM ZUSAMMENHANG MIT TUBERKULOSE
PROCÉDÉS RELATIFS À LA TUBERCULOSE

(30) Priority: 21.05.2019 GB 201907157
(43) Date of publication of application: 30.03.2022
(73) Proprietor: PBD Biotech Limited, Birmingham, West Midlands B3 1RB (GB)
(72) Inventor: SWIFT, Benjamin, Birmingham, West Midlands B3 1RB (GB); REES, Catherine, Nottingham Nottinghamshire NG7 2RD (GB)
(74) Representative: Stratagem IPM Limited
(86) International application number: PCT/GB2020/050524
(87) International publication number: WO 2020/234555

(56) References cited:
- WO-A1-2015/049516
- US-A1- 2008 171 345
- US-A1- 2011 021 367
- US-A1- 2011 104 194
- US-A1- 2015 192 593
- US-A1- 2017 003 286
- J. M. ACHKAR ET AL: "Incipient and Subclinical Tuberculosis: Defining Early Disease States in the Context of Host Immune Response", JOURNAL OF INFECTIOUS DISEASES. JID, vol. 204, no. suppl 4, 13 October 2011 (2011-10-13), US, pages S1179 - S1186, XP055660274, ISSN: 0022-1899, DOI: 10.1093/infdis/jir451
- PAUL K. DRAIN ET AL: "Incipient and Subclinical Tuberculosis: a Clinical Review of Early Stages and Progression of Infection", CLINICAL MICROBIOLOGY REVIEWS., vol. 31, no. 4, 1 January 2018 (2018-01-01), US, pages e00021 - 18, XP055660283, ISSN: 0893-8512, DOI: 10.1128/CMR.00021-18
- ELISA PETRUCCIOLI ET AL: "Correlates of tuberculosis risk: predictive biomarkers for progression to active tuberculosis", EUROPEAN RESPIRATORY JOURNAL, vol. 48, no. 6, 1 December 2016 (2016-12-01), GB, pages 1751 - 1763, XP055660342, ISSN: 0903-1936, DOI: 10.1183/13993003.01012-2016
- SARA SULIMAN ET AL: "Four-Gene Pan-African Blood Signature Predicts Progression to Tuberculosis", AMERICAN JOURNAL OF RESPIRATORY AND CRITICAL CARE MEDICINE., vol. 197, no. 9, 1 May 2018 (2018-05-01), US, pages 1198 - 1208, XP055591229, ISSN: 1073-449X, DOI: 10.1164/rccm.201711-2340OC
- PETERS JULIAN S ET AL: "Advances in the understanding of Mycobacterium tuberculosis transmission in HIV-endemic settings", LANCET INFECTIOUS DISEASES, vol. 19, no. 3, 1 March 2019 (2019-03-01), XP085614659, ISSN: 1473-3099, DOI: 10.1016/S1473-3099(18)30477-8
- K. K. SINGH ET AL: "Immunogenicity of the Mycobacterium tuberculosis PPE55 (Rv3347c) Protein during Incipient and Clinical Tuberculosis", INFECTION AND IMMUNITY, vol. 73, no. 8, 1 August 2005 (2005-08-01), US, pages 5004 - 5014, XP055660322, ISSN: 0019-9567, DOI: 10.1128/IAI.73.8.5004-5014.2005
- AJAY WANCHU ET AL: "Biomarkers for Clinical and Incipient Tuberculosis: Performance in a TB-Endemic Country", PLOS ONE, vol. 3, no. 4, 30 April 2008 (2008-04-30), pages e2071, XP055408435, DOI: 10.1371/journal.pone.0002071
- VERMA RAMAN ET AL: "A novel high sensitivity bacteriophage-based assay identifies low level M. tuberculosis bacteraemia in immunocompetent patients with active and incipient TB", CLINICAL INFECTIOUS DISEASES, OXFORD UNIVERSITY PRESS, US, 22 June 2019 (2019-06-22), pages 1 - 4, XP009518330, ISSN: 1537-6591, DOI: 10.1093/CID/CIZ548

## Description

### Field of the Invention

This invention relates to methods of characterising a tuberculosis (TB) disease state in a subject, such that an individual with incipient TB, asymptomatic or latent TB infection (LTBI) may be identified. The invention further relates to methods of treatment, methods of improving tuberculosis prognosis, and kits for use in assessment of tuberculosis disease state in a subject.

### Background of the Invention

One-third of the world's population is estimated to be infected with *Mycobacterium tuberculosis* (Mtb), the organism responsible for tuberculosis (TB) disease. Worldwide, it was estimated that 10 million people developed TB disease in 2017. Cases of TB disease occur in all countries and age groups, but the highest prevalence is in adults in India, China, Indonesia, the Philippines, Pakistan, Nigeria, Bangladesh, and South Africa. In the same year, there were an estimated 1.3 million deaths attributable to TB among HIV-negative people, and an additional 300,000 deaths among HIV-positive people. TB is the leading cause of death from a single infectious agent in humans. In 2017, the proportion of people with TB who died from the disease was 16%, down from 23% in 2000, but a long way from the 10% rate necessary to meet the World Health Organization (WHO) End TB Strategy 2020 target (Global Tuberculosis Report 2018, World Health Organization; https://www.who.int/tb/publications/global_report/en/).

The traditional understanding of TB disease is binary; in one state, latent TB infection (LTBI), defined as a state of persistent immune response to *M. tuberculosis* without clinically manifested evidence of active TB disease, the patient is asymptomatic and replication of *M. tuberculosis* is at a low level and controlled by the immune system. Latent TB infection gives *M. tuberculosis* the ability to persist undetected within populations. An individual may have a latent TB infection for many years without it being detected. Current estimates of the number of people worldwide with a latent TB infection range from 1.7 billion to more than 2 billion (23 - 27 % of the world's population). In contrast, in the active TB disease state the patient is symptomatic due to the pathology resulting from uncontrolled *M. tuberculosis* replication.

Only a proportion of individuals with LTBI will go on to develop active TB disease. The lifetime risk of developing active TB amongst infected individuals is 5-15%, with the highest risk in the first 2 years after infection. Clinically latent infection appears to represent a spectrum of outcomes, from presumably cleared infection to subclinical disease without overt symptoms. Recent evidence supports the existence of a transitional state of human Mtb infection, termed incipient TB, that is clinically latent but characterised by increased risk of progression to active TB in the absence of further intervention, and a host blood transcriptional profile that overlaps with disease (Cobelens F, et al., From latent to patent: rethinking prediction of tuberculosis. The Lancet Respiratory Medicine 2017; 5(4): 243-4; Zak DE, et al., A blood RNA signature for tuberculosis disease risk: a prospective cohort study. Lancet 2016; 387: 2312-22). Individuals with incipient TB have not yet developed induced clinical symptoms, radiographic abnormalities, or microbiologic evidence consistent with active TB. Biomarkers of incipient tuberculosis are discussed in (Achkar J.M. et al., Incipient and Subclinical Tubercuolosis: defining early disease states in the context of host immune response; Journal of Infectious diseases, vol. 204, no. suppl. 4, pages S1179-S1186 (2011) and Drain P.K. et al., Incipient and Subclinical Tuberculosis: a clinical review of early stages and progession of infection; Clinical Microbiology Reviews, vol. 31, no. 4, pages e00021-18 (2018))

Currently, treatment of LTBI is the main treatment intervention available to prevent development of active TB disease in those already infected with Mtb. TB preventive treatment for LTBI is expanding, but most of those for whom it would be beneficial are not yet accessing care. One reason for this is under-diagnosis of people with LTBI, for multiple reasons including lack of or subclinical symptoms meaning people do not seek out health care, and failure to accurately test for TB when people do present to health professionals.

There is a need therefore for a rapid, simple, accurate test which allows for the detection of incipient TB at a time point early enough to permit effective intervention and treatment. It is against this background that the present invention was developed.

### Summary of the Invention

In a first aspect of the invention there is provided:
a method of diagnosis of a disease in an asymptomatic human subject, the method comprising:
obtaining a sample of peripheral blood mononuclear cells (PBMCs) isolated from the subject;
admixing Mycobacteria-specific bacteriophage D29 with the sample of PBMCs to produce an admixture;
incubating the admixture under conditions that permit lysis of Mycobacteria;
isolating DNA from the admixture;
determining if a Mycobacterial DNA sequence is present or absent in the DNA isolated from the admixture by one or more of: performing PCR on the DNA isolated from the admixture using forward and reverse primers specific for the Mycobacterial DNA sequence, and/or performing sequencing suitable to identify the Mycobacterial DNA sequence, and optionally determining homology between the Mycobacterial DNA sequence, if present, and known Mycobacterial DNA sequences;
performing a Mycobacterium-specific cell-mediated immune (CMI) response test on a sample isolated from the subject to generate a positive or negative CMI response test outcome;
wherein the asymptomatic human subject is diagnosed as having incipient tuberculosis (TB) when the Mycobacterial DNA sequence is present, with either a positive or negative CMI response test outcome, where present; and
wherein the asymptomatic human subject is diagnosed as having latent tuberculosis infection (LTBI) when the Mycobacterial DNA sequence is absent and a positive CMI response test outcome is present.

In another aspect of the invention there is provided:
a method of diagnosing incipient tuberculosis (TB) in a human subject, the method comprising:
obtaining a sample of peripheral blood mononuclear cells (PBMCs) isolated from the subject;
admixing a Mycobacteria-specific bacteriophage with the sample of PBMCs to produce an admixture;
incubating the admixture under conditions that permit lysis of Mycobacteria;
isolating DNA from the admixture;
determining if one or more Mycobacterial DNA sequence is present in the DNA isolated from the admixture;
wherein the presence of a Mycobacterial DNA sequence indicates that the subject has incipient tuberculosis.

In one embodiment the human subject is an asymptomatic human subject, i.e. the subject is not displaying any symptoms of active tuberculosis disease.

In another aspect of the invention there is provided:
a method of predicting if a subject is at risk of developing or having incipient tuberculosis (TB), the method comprising:
obtaining a sample of peripheral blood mononuclear cells (PBMCs) isolated from the subject;
admixing a Mycobacteria-specific bacteriophage with the sample of PBMCs to produce an admixture;
incubating the admixture under conditions that permit lysis of Mycobacteria;
isolating DNA from the admixture;
identifying if Mycobacterial DNA sequences are present in the DNA;
wherein the presence of Mycobacterial DNA sequences indicates that the subject is at risk of developing or having incipient TB.

In another aspect of the invention there is provided:
a method of predicting if a subject is at risk of developing active tuberculosis (TB), the method comprising:
obtaining a sample of peripheral blood mononuclear cells (PBMCs) isolated from the subject;
admixing a Mycobacteria-specific bacteriophage with the sample of PBMCs to produce an admixture;
incubating the admixture under conditions that permit lysis of Mycobacteria;
isolating DNA from the admixture;
identifying if Mycobacterial DNA sequences are present in the DNA;
wherein the presence of Mycobacterial DNA sequences indicates that the subject is at risk of developing active TB.

In another aspect of the invention there is provided:
a method of assessing the tuberculosis (TB) disease prognosis for a subject, the method comprising:
obtaining a sample of peripheral blood mononuclear cells (PBMCs) isolated from the subject;
admixing a Mycobacteria-specific bacteriophage with the sample of PBMCs to produce an admixture;
incubating the admixture under conditions that permit lysis of Mycobacteria;
isolating DNA from the admixture;
identifying if Mycobacterial DNA sequences are present in the DNA isolated from the admixture;
wherein the presence of Mycobacterial DNA sequences indicates that the subject is at risk of having incipient TB and/or developing active TB disease.

In another aspect of the invention there is provided:
a composition comprising one or more anti-tuberculosis medicament for use in a method of treating tuberculosis (TB) in a subject in need thereof, the method comprising:
obtaining a sample of peripheral blood mononuclear cells (PBMCs) isolated from the subject;
using a bacteriophage-mediated Mycobacterial DNA release assay to identify the presence of incipient TB in the subject; and, wherein the composition is administered to the subject where incipient TB is identified.

A bacteriophage-mediated Mycobacterial DNA release assay means any assay or method as described herein whereby a Mycobacterium-specific bacteriophage (i.e. a bacteriophage that will selectively lyse mycobacteria or a particular mycobacterium species or strain) is used to lyse Mycobacteria in a sample in order to release the Mycobacterial DNA, following which the presence of Mycobacterial DNA is confirmed in the sample by any suitable method, for example PCR using Mycobacterium-specific primers, or by next-generation sequencing.

In another aspect of the invention there is provided:
a composition comprising one or more anti-tuberculosis medicament for use in a method of treating incipient tuberculosis (TB) in a subject in need thereof, the method comprising:
obtaining a sample of peripheral blood mononuclear cells (PBMCs) isolated from the subject;
admixing a Mycobacteria-specific bacteriophage with the sample of PBMCs to produce an admixture;
incubating the admixture under conditions that permit lysis of Mycobacteria;
isolating DNA from the admixture;
identifying the presence of Mycobacterial sequences in the DNA; and
administering the compound to the subject where Mycobacterial sequences are identified in the DNA.

In another aspect of the invention there is provided:
a composition comprising one or more anti-tuberculosis medicament for use in a method of treating tuberculosis comprising improving tuberculosis (TB) prognosis in a subject, the method comprising:
obtaining a sample of peripheral blood mononuclear cells (PBMCs) isolated from the subject;
using a bacteriophage-mediated Mycobacterial DNA release assay to identify the presence of incipient TB in the subject; and, wherein the composition is administered to the subject where incipient TB is identified.

Suitable anti-TB medicaments or therapies in accordance with any aspect of the invention will be known to those skilled in the art, and may be selected based on clinical or other indication. Suitable anti-TB medicaments or therapies may include rifampicin, ethambutol hydrochloride, pyrazinamide, and isoniazid (with pyridoxine hydrochloride), which may be administered to a subject alone (one anti-TB medicament or therapy) or in any suitable combination of more than one anti-TB medicament or therapy.

In an embodiment in accordance with any aspect of the invention, the sample of peripheral blood mononuclear cells (PBMCs) may be isolated from the subject by any suitable method, for example density centrifugation (e.g. Ficoll-Paque), isolation by cell preparation tubes (CPT^{™}), isolation by SepMate^{™} tubes, or isolation using an erythrocyte aggregation agent e.g. HetaSep^{™}.

In another aspect of the invention there is provided:
use of a bacteriophage for measuring in a blood sample isolated from a human subject the presence of one or more Mycobacterial biomarker for assessing if said human subject is at risk of developing or has incipient tuberculosis (TB) or active tuberculosis disease, optionally, in the form of a kit.

In one embodiment in accordance with any aspect of the invention, determining if one or more Mycobacterial DNA sequence is present in the DNA may comprise performing polymerase chain reaction, for example using Mycobacterium-specific primers.

In one embodiment in accordance with any aspect of the invention, the one or more Mycobacterial DNA sequence may comprise e.g. the IS611 0 element and/or the IS900 element.

In one embodiment in accordance with any aspect of the invention, the method may comprise sequencing the one or more Mycobacterial DNA sequence, and optionally determining homology between the Mycobacterial DNA sequence and known Mycobacterial DNA sequences. Known mycobacterial DNA sequences may comprise those identified from published sequence databases, or they may comprise DNA sequences identified from TB-infected individuals that the subject is known or is suspected to have come into contact with.

In one embodiment in accordance with any aspect of the invention, the subject may be a human subject. In one embodiment in accordance with any aspect of the invention, the subject may be a mammal, such as but not limited to a primate a great ape or a human. In one embodiment in accordance with any aspect of the invention, the subject may be selected from at least one of: an asymptomatic subject and/or a TB-contact subject. In one embodiment in accordance with any aspect of the invention, the subject may be selected from at least one of: an asymptomatic human subject and/or a TB-contact human subject. An asymptomatic subject means any subject who is not displaying any symptoms of active tuberculosis disease that are typically used to confirm diagnosis. Such symptoms may be determined by clinical or radiological assessment. Additionally the subject may give a negative test result by sputum smear microscopy and/or mycobacterial culture. A TB-contact subject means any subject who has come into contact with another individual known or suspected to have a tuberculosis infection. A TB-contact subject may be identified by TB contact tracing.

In one embodiment in accordance with any aspect of the invention, the subject may be immunocompromised and/or immunosuppressed. Both immunocompromise and immunosuppression may be considered states of immunodeficiency. Immunocompromise in a subject may be caused by a disease that directly or indirectly causes immunosuppression. Examples of such diseases include particular cancers (e.g. leukaemia, lymphoma, multiple myeloma, cancers of bone marrow, cancers of blood cells), chronic infections e.g. acquired immunodeficiency syndrome (AIDS) caused by infection with the human immunodeficiency virus (HIV), and various hormonal and metabolic disorders including anaemia, hypothyroidism and hyperglycaemia. In one embodiment, the subject may be infected with HIV. Immunosuppression in a subject may also be caused by malnutrition, aging, the administration of an immunosuppressive agent, medication or immunosuppressant (for example chemotherapy, a disease-modifying anti-rheumatic drug (a DMARD), an immunosuppressive drug administered in relation to organ transplant (an antirejection medication), a steroid including a corticosteroid e.g. a glucocorticoid), exposure to environmental toxins, or the abuse of alcohol, illegal drugs or nicotine. In one embodiment, the subject may be taking or have taken immunosuppressive medication, particularly one or more medications selected from the group consisting of: chemotherapy, a DMARD, an antirejection medication and a glucocorticoid.

In one embodiment in accordance with any aspect of the invention, the subject may be a TB-contact subject, and suitably the method is performed on a sample of PBMCs obtained from the subject within 12 months, 6 months, 5 months, 4 months, 3 months, 2 months or 1 month of the subject coming into contact with a TB-infected individual.

In one embodiment in accordance with any aspect of the invention, the bacteriophage may be (myco)bacteriophage D29 or TM4, preferably D29. Other bacteriophages suitable for lysing Mycobacteria will be known to those skilled in the art, e.g. see Hatfull GF. (2018) Mycobacteriophages. Microbiol Spectr. 2018;6(5):10.1128/microbiolspec.GPP3-0026-2018. doi:10.1128/microbiolspec.GPP3-0026-2018.

In one embodiment in accordance with any aspect of the invention, incubating the admixture under conditions that permit lysis of Mycobacteria may comprise incubation at approximately 37 °C for approximately or less than 6 hours, 5 hours, 4 hours, 3.5 hours, 3 hours, 2.5 hours, 2 hours, 1.5 hours or 1 hour, preferably for approximately 3.5 hours.

In one embodiment in accordance with any aspect of the invention, the optional CMI response test may comprise an interferon-gamma release assay (IGRA) test. An example of a suitable IGRA test is the QuantiFERON-TB Gold Plus ((QFT) Qiagen Inc) assay. Other Mycobacteria-specific CMI response tests will be known to those skilled in the art.

Various further aspects and embodiments of the present invention will be apparent to those skilled in the art in view of the present disclosure.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

It will further be appreciated by those skilled in the art that although the invention has been described by way of example with reference to several embodiments. It is not limited to the disclosed embodiments and that alternative embodiments could be constructed without departing from the scope of the invention as defined in the appended claims.

Unless specifically stated, a process comprising steps may be performed in any suitable order. Thus steps can be performed in any appropriate order.

Sequence homology can be measured using known methods. For example the UWGCG Package provides the BESTFIT program which can be used to calculate homology (for example used on its default settings) (Devereux et al (1984) Nucleic Acids Research 12, 387-395). The PILEUP and BLAST algorithms can be used to calculate homology or to line up sequences (typically on their default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information (http://www.ncbi.nlm.nih.gov/).

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

### Brief Description of the Figures

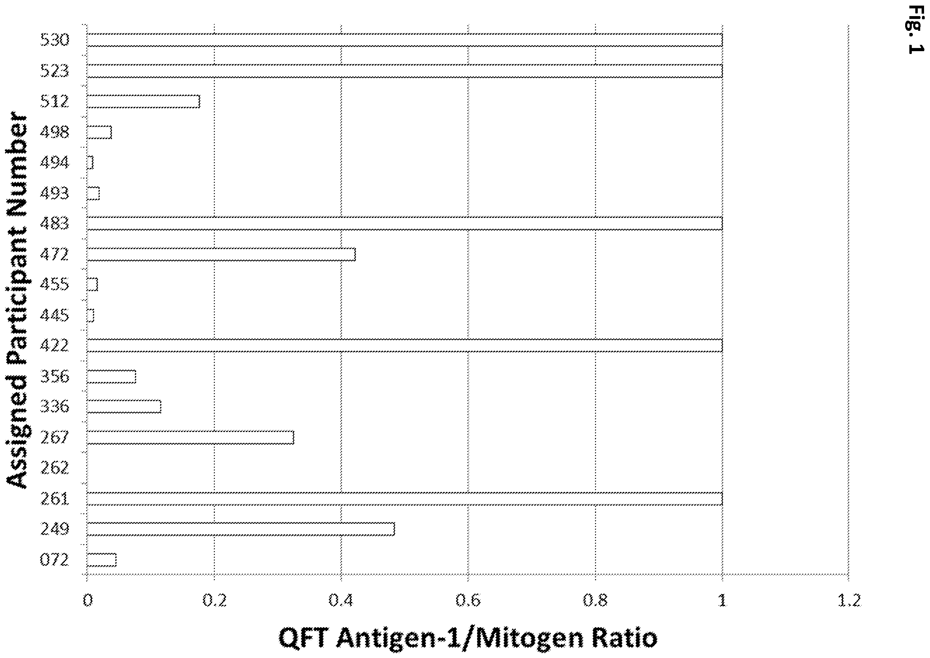

**Figure 1** is a graph showing the results of a Mycobacterium-specific cell-mediated immune (CMI) response test (an IGRA test: the QuantiFERON-TB Gold Plus ((QFT) Qiagen Inc) assay) at baseline for a number of asymptomatic subjects determined to be in group 2 during the course of the study. All the subjects included in Fig. 1 showed a positive QFT test outcome at one or more timepoint during the study, and all were asymptomatic at baseline. The Y axis shows an arbitrary assigned participant number for each subject, the X axis shows the ratio between Antigen-1 and Mitogen (positive control antigen) as determined using the QFT kit. Subjects corresponding to assigned participant numbers 455, 493 and 494 tested positive by bacteriophage-mediated Mycobacterial DNA release assay (Actiphage^{™}) at baseline.

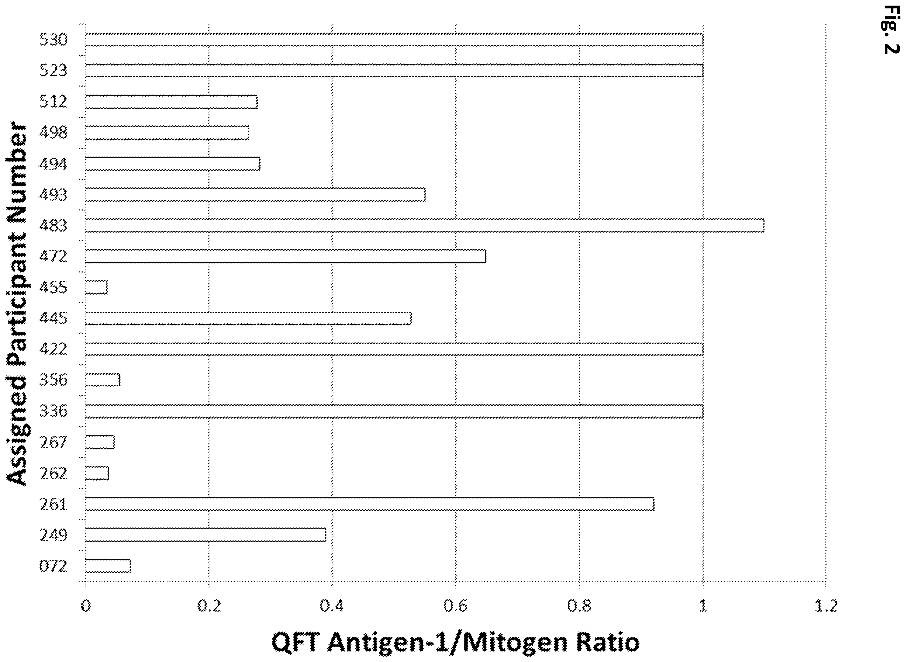

**Figure 2** is a graph showing the results of the QuantiFERON-TB Gold Plus ((QFT) Qiagen Inc) assay at the 3-month (approx. 8-12 weeks) timepoint for a number of asymptomatic subjects determined to be in group 2 during the course of the study. All the subjects included in Fig. 2 showed a positive QFT test outcome at the 3-month timepoint at least, and all were asymptomatic at baseline and 3 months. The Y axis shows an arbitrary assigned participant number for each subject, the X axis shows the ratio between Antigen-1 and Mitogen (positive control antigen), as determined using the QFT kit. Subjects corresponding to assigned participant numbers 455, 493 and 494 tested positive by QFT and by bacteriophage-mediated Mycobacterial DNA release assay (Actiphage^{™}) at the 3 month timepoint.

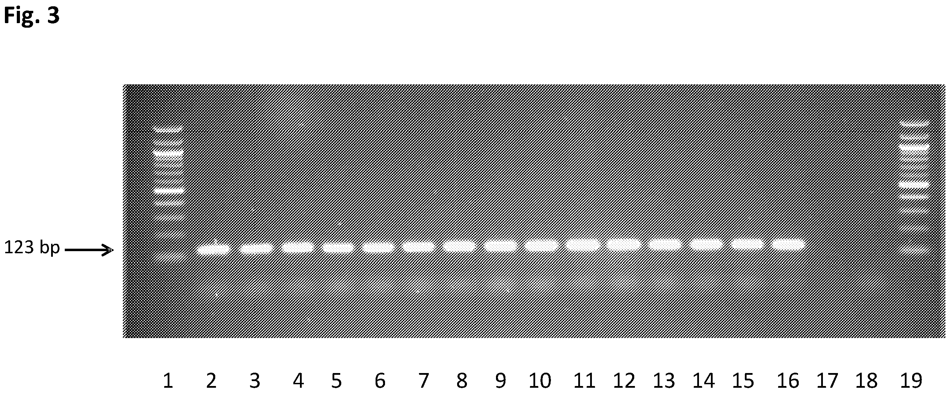

**Figure 3** is an image of a gel showing PCR products generated using Mycobacterium-specific primers designed to amplify a 123 bp region of the IS6110 element. Lanes 1 and 19: NEB 100 bp DNA ladder, Lanes 2 - 15: subject samples prepared using the bacteriophage-mediated Mycobacterial DNA release assay (Actiphage^{™}) method, with Lane 3 and Lane 10 representing the sample obtained from participant number 455, with PBMCs prepared by either Ficoll or Hetasep, respectively. Lane 16 - PCR Positive Control (1 µL of BCG DNA), Lane 17 - Blank, Lane 18 - PCR Negative control (no template, water control).

### Detailed Description of the Invention

Tuberculosis (TB) is the leading cause of death in humans from a single infectious agent. The causative organism, *M. tuberculosis* (Mtb) persists within populations by establishing asymptomatic latent infection (LTBI); a reservoir for future disease. It is estimated that 5-10% of the LTBI population will develop TB prospectively, usually within a 2-year period of acquiring infection. The patho-biological mechanisms underpinning progression to and severity of active TB are poorly understood.

The World Health Organization has identified that tests that are highly predictive of development of TB disease in the near future are urgently needed. Ideal tests would offer significantly increased predictive value for the development of active TB disease among infected individuals than the currently available tests for LTBI, in order to meet WHO disease treatment and eradication targets (Consensus meeting report: development of a Target Product Profile (TPP) and a framework for evaluation for a test for predicting progression from tuberculosis infection to active disease. Geneva: World Health Organization; 2017 (WHO/HTM/TB/2017.18)).

Previously available diagnostic tests are not sufficiently sensitive or specific to ensure accurate identification of all cases of LTBI. Furthermore, the tests poorly predict whether an individual with LTBI will progress to active TB in the future (i.e. whether the individual has incipient TB). This translates into a high number of individuals who would need to be treated in order to prevent one case of active TB and as such is a further barrier to expansion of the programmatic management of TB.

Previously adopted diagnostic tests for LTBI, such as interferon-gamma (IFN-y) release assays (IGRAs) or tuberculin skin tests (TST, e.g. the Mantoux test), measure the T cell response to *Mycobacterium tuberculosis,* and therefore indirect evidence of *M. tuberculosis* exposure. Tests that rely on patient immune response can by definition not yield results until a measureable T cell response has been induced, such response taking time to develop (at least 6 weeks post infection). The resultant delay in diagnosis allows a greater amount of time for bacterial replication before diagnosis and therefore before treatment can occur, leading to reduced prognosis for patients and an increased likelihood of transmission within communities. Additionally, this immune memory response is reduced in ageing, immunocompromised or immunosuppressed patients, thereby leading to inaccurate (false negative or indeterminate) test results. For example subjects receiving immunosuppressive agents can have false-negative TST results (Agarwal S. et al., 2014, Steroids Decrease Prevalence of Positive Tuberculin Skin Test in Rheumatoid Arthritis: Implications on Anti-TNF Therapies. Interdisciplinary Perspectives on Infectious Diseases, 2014(5759):430134) or false-negative or indeterminate IGRA results (Belliere and Blancher, 2017, QuantiFERON test interpretation in patients receiving immunosuppressive agents: an alert. European Respiratory Journal Apr 2017, 49 (4) 1602102; DOI: 10.1183/13993003.02102-2016). Testing based on the immune response to Mtb also presents the possibility of falsely identifying as at risk of developing active TB disease those individuals in whom the infection has been effectively cleared by the immune system, or those who have been previously vaccinated against Mtb (false positives). Research has shown that the positive predictive value of the tuberculin skin test and the interferon-γ release assays to predict active TB disease occurring within two years is 1.5 and 2.7% respectively (Consensus meeting report: development of a Target Product Profile (TPP) and a framework for evaluation for a test for predicting progression from tuberculosis infection to active disease. Geneva: World Health Organization; 2017 (WHO/HTM/TB/2017.18)).

Furthermore, the current testing methods have operational requirements that make them impractical or too expensive for use in remote locations or those countries where TB poses the greatest challenges. Additionally, some TB diagnostic tests require the production of sputum by a subject, which is not generally possibly in asymptomatic individuals.

### Incipient TB

Incipient TB describes a recently recognised classification of tuberculosis in which individuals are asymptomatic, but characterised by increased risk of progression to active TB in the absence of further intervention in comparison with LTBI individuals. Individuals with incipient TB have not yet developed induced clinical symptoms, radiographic abnormalities, or microbiologic evidence consistent with active TB (Drain, P et al. 2018. Incipient and Subclinical Tuberculosis: a Clinical Review of Early Stages and Progression of Infection. Clin Microbio Rev. 31 (5): e00021-18). The postulate that there might be a prolonged asymptomatic phase of early disease during which pathology evolves prior to clinical presentation with active disease is now widely accepted. This state identifies incipient tuberculosis. Some individuals with incipient tuberculosis might not progress to active disease for 12 months or longer.

Incipient TB had been challenging to diagnose to date because there was no satisfactory positive test for this disease state that distinguishes it from latent TB (LTBI; asymptomatic TB that is not likely to develop into active TB due to effective clearance or containment by the immune system). Traditionally, diagnosis of incipient TB relies on a positive test usually associated with LTBI, such as IGRA, viewed in combination with an initial absence of clinical or radiological symptoms that is then replaced with the presence of one or more clinical or radiological symptom, or positive microbiological test result, as active TB develops. This means that incipient TB is generally only diagnosable with hindsight, after active TB symptoms have already emerged. This inability to distinguish incipient TB from LTBI before active TB disease symptoms develop can result in unnecessary treatment of individuals who are unlikely to ever develop active TB (i.e. those with LTBI not incipient TB), and/or potentially delayed treatment of those with incipient TB, meaning increased likelihood of transmission and reduced prognosis for those patients.

### LTBI testing

### Interferon-v release assay

Interferon-γ (interferon-gamma) release assays (IGRAs) are examples of cell-mediated immune (CMI) response tests, and are tests used in the diagnosis of some infectious diseases, especially tuberculosis. Interferon-γ (IFN-γ) release assays rely on the fact that T-lymphocytes will release IFN-γ when exposed to specific antigens.

One example of an IFN-γ assay is the QuantiFERON-TB Gold Plus ((QFT) Qiagen Inc) assay which quantitates the amount of IFN-γ produced in response to the ESAT-6 and CFP-10 antigens from Mycobacterium tuberculosis. The result is reported as an ELISA absorbance or a calculated IFN-γ level in IU/ml.

Another example of an IGRA is the T-SPOT.TB assay (Oxford Immunotec) which is an enzyme-linked immunosorbent spot (ELISPOT) assay performed on peripheral blood mononuclear cells that are incubated with ESAT-6 and CFP-10 peptides. The result is reported as the number of IFN-γ-producing T cells. An individual is considered positive for Mycobacterium tuberculosis infection if the spot counts in the TB antigen wells exceed a specific threshold relative to the negative control wells.

### Mantoux tuberculin skin test

The Mantoux test (tuberculin skin test; PPD test) is a tool for screening for TB infection reliant on the immune memory response against an intradermal injection of tuberculin, a glycerol extract derived from cultures of *Mycobacterium tuberculosis.* T-cells sensitized by prior infection are recruited to the skin site where they release lymphokines inducing an induration (a discrete, pale elevation of the skin) of 6 to 10mm in diameter 48-72hrs later.

The currently adopted tests for LTBI (such as interferon-γ release assays or the tuberculin skin test) for *M. tuberculosis* infection are imperfect as incipient TB tests (ITT) for three main reasons:
1. They measure *M. tuberculosis* infection indirectly; that is they measure the T cell response to *M. tuberculosis.* This is a delayed response following Mtb infection (it can take approximately 6 weeks to develop in humans), meaning that at the point of positive testing of a recently-infected individual, the bacterium will have had time to replicate, thereby reducing prognosis for an individual and increasing the potential for transmission to others. Furthermore, these indirect tests cannot distinguish between vaccinated, previously infected and currently infected individuals.
2. They are insensitive to certain subgroups of the population. Individuals with a weakened immune response such as ageing individuals, immunocompromised individuals (such as HIV infected), or individuals taking immunosuppressive drugs will have a reduced ability to mount a T cell response and therefore are less likely to generate a positive test result even if they are infected with *M. tuberculosis.*
3. LTBI tests poorly predict whether an individual will progress to active TB in the future.

The WHO has set out its expectations for a clinically useful incipient TB test (ITT) Consensus meeting report: development of a Target Product Profile (TPP) and a framework for evaluation for a test for predicting progression from tuberculosis infection to active disease. Geneva: World Health Organization; 2017 (WHO/HTM/TB/2017.18). These state that such tests should be considered rule-in tests wherein a negative result provides limited information but a positive result indicates that active TB disease will probably develop. ITTs would ideally be used for screening of those who have been recently exposed to MTB, such as contacts of infectious tuberculosis patients (TB-contacts). TB contact tracing is a process used to stop the spread of TB in the community. It involves finding and informing the people that an infected person has been in contact with (TB contacts) so they can get counselling, testing and treatment if necessary. An ideal ITT would have characteristics that would allow scale-up of contact tracing strategies to facilitate mass test-and-treat campaigns.

Characteristics of an ideal incipient TB test:
- To be negative in individuals never exposed to TB, including individuals who may be symptomatic for other (respiratory) illnesses but who have an alternative diagnosis.
- To be negative in individuals who are infected with Mtb but who do not have incipient TB. They might have a persistent TB infection, have a positive LTBI test (TST or IGRA) but do not develop TB disease within the next 2 years.
- To be negative in individuals who have been treated for LTBI.
- To be positive in individuals who develop TB within a short period after the test was done (e.g. 2 years), and who do not have any indication of re-exposure after the test was performed.
- To be positive in individuals with symptomatic TB disease.
- To be negative in individuals who completed TB treatment and are considered cured.

WHO defined operational characteristics for incipient TB tests:

| Operational characteristics | Optimal | Minimal |
|---|---|---|
| No. of steps to be performed by operator | < 2, no timed steps | < 10, 1-2 timed steps |
| Volume measurements | None | Measuring device provided with kit |
| Sample preparation | None or fully integrated | Allows for centrifugation/ incubation |
| Data analysis | Integrated | Integrated |
| Time to results | < 24 hours | 2-5 days |
| Biosafety | Universal precautions | Universal precautions, biosafety Level II |
| Operating Temperature | Between 5 and 50 °C, 90% humidity | Between 5 and 30 °C, 70% humidity |
| Reagents | Self-contained within test kit | Up to 2 external reagent, reconstitution not required |
| Stability of test kit / reagent | 24 months at 40 °C, 90% humidity, should be able to tolerate stress during transport (3 days at 50 °C) | 12 months at 30 °C, 70% humidity, cold chain required for transport |
| Instrumentation | Preferably instrument free. If instrument: Small, portable or handheld instrument (<1 kg) that can operate on battery or solar in places with interrupted power supply | Centralized testing platform suitable for use in laboratories. |
| Waste disposal | Standard infected waste disposal at health center | Standard infected waste disposal at health center |

The development by the present inventors of a method to detect viable mycobacteria in body fluids using phage combined with DNA amplification (bacteriophage-mediated Mycobacterial DNA release assay) has been previously described in WO2015049516, and its utility in demonstrating low-grade *M. bovis* bacteraemia (less than 10² cells per ml) in the blood of infected cattle has been demonstrated (Swift BM et al., Evidence of Mycobacterium tuberculosis complex bacteraemia in intradermal skin test positive cattle detected using phage-RPA. Virulence 2016; 7(7): 779-88).

The bacteriophage-mediated Mycobacterial DNA release assay method (Actiphage^{™}) has now been adapted for detecting human Mtb infection. The outcomes of a proof-of-concept study applying the highly sensitive bacteriophage-mediated Mycobacterial DNA release assay Actiphage^{™} method to well-characterised clinical cohorts is herein described. The incipient TB detection method described herein offers a number of advantages over the currently-adopted tests for LTBI, including increased specificity (probability that a test result will be negative when the disease is not present) and sensitivity (probability that a test result will be positive when the disease is present) for patients likely to develop active TB disease, improved false positive reporting, speed to results (less than 24 hours), and no requirement for cold chain transport. These are all highly advantageous attributes in accordance with the WHO defined characteristics for ideal incipient TB tests.

The early diagnostic potential of the method disclosed in detecting human subjects with incipient TB, i.e. those asymptomatic individuals (including those who might previously have been identified as having LTBI) at risk of developing active TB disease, is described. It is demonstrated that incipient human TB is associated with, and may be identified by, detecting viable Mycobacteria in the blood (e.g. Mtb bacteraemia) during early infection.

The evidence for TB-associated Mtb bacteraemia, even during active TB, is unclear. Previous studies applying culture and nucleic acid amplification tests (NAAT) to blood samples from patients with active TB have been disappointing (Shenai S. et al. Exploring alternative biomaterials for diagnosis of pulmonary tuberculosis in HIV-negative patients by use of the GeneXpert MTB/RIF assay. J Clin Microbiol 2013; 51(12): 4161-6). Improved Mtb detection has been reported for active TB disease of greater severity and following analysis of higher blood volumes, suggesting these methods are limited by inadequate sensitivity. The Mtb blood titre during active TB disease is expected to be significantly higher than during the early, asymptomatic phases of tuberculosis infection (i.e. soon after contact with/ infection from an infected individual), therefore it is unexpected that testing for viable Mtb in the blood should be an effective determinant of incipient TB or prognostic for subsequent active TB disease. Without wishing to be bound by any particular theory, the inventors postulate that the relative inaccessibility of intracellular Mtb DNA within circulating PBMCs is an important contributory factor to the inadequate sensitivity of other Mtb detection methods. The approach described herein overcomes this by phage-mediated lysis of intracellular Mtb, thereby efficiently releasing bacterial DNA for sensitive NAAT in a second-step. The approach described herein offers the surprising advantage that even subjects in the early stages of TB infection, where the titre of circulating mycobacteria in the blood may be very low, can be effectively identified. The advantages of a rapid, low-cost assay that offers a positive, microbiological diagnosis, in the absence of sputum, are clear

### Examples

### Example 1

HIV sero-negative adult patients (age ≥ 18 years) were recruited into 4 groups according to the following criteria (see Table 1 for further data):
1. Active TB disease (pulmonary TB; PTB) - based on positive Xpert-Ultra (Xpert MTB/RIF Ultra, Cepheid Inc) or Mtb culture from respiratory tract samples, in patients with supporting clinical and radiological disease characteristics (n=15)
2. Asymptomatic recent PTB contacts identified at contact tracing, with a positive QuantiFERON-TB Gold Plus ((QFT) Qiagen Inc) test and normal chest X-ray (n=18)
3. Non-TB acute respiratory illness control group - patients initially referred to the TB service with symptoms that indicated suspected PTB, but who were subsequently diagnosed with a non-TB illness and confirmed negative by microbiological testing for Mtb (n=5)
4. Healthy control group - asymptomatic and QFT negative participants with no history of previous TB contact (n=28)

All participants provided blood samples for Actiphage^{™} bacteriophage-mediated Mycobacterial DNA release assay testing for bacteraemia of viable Mycobacteria on recruitment, and received 12-months prospective clinical follow-up. In addition, group 2 had QFT and Actiphage^{™} testing repeated after 8-12 weeks to capture IGRA (QFT) seroconversion events (those participants who tested negative by QFT at the first time point but positive at the second time point, presumed to be due to delayed T cell response/delayed detectability of IFN-y). Group 2 participants were retained in the study if QFT-positive at the second time-point; none of these participants received chemoprophylaxis. Active PTB patients (group 1) were sampled prior to commencing anti-tuberculous treatment.

Clinical and laboratory teams were blinded to the outcome of the Actiphage^{™} bacteriophage-mediated Mycobacterial DNA release assay testing and the study groups from which samples originated, respectively, until the end of the study. Ethics approval was provided by the regional Research and Ethics Committee (REC 15/EM/0109) and all participants provided written informed consent at enrolment.

QFT and Xpert-Ultra testing were conducted according to the manufacturers' instructions, and Mtb culture was conducted according to standard methods. A negative Mtb culture result means no bacterial growth occurred following an 8 week incubation period.

QFT ELISA data for participants determined as asymptomatic recent PTB contacts (group 2) is illustrated in Figures 1 and 2 (at baseline and 3 months respectively).

For the bacteriophage-mediated Mycobacterial DNA release assay (Actiphage^{™}) test, blood (5 ml) was collected into sodium heparin tubes (Sarstedt) and stored at room temperature until processing. PBMCs were isolated from 2 ml blood aliquots according to the manufacturer's instructions by one of two alternative methods: i. Ficoll-Paque Plus (GE Healthcare) using Leucosep tubes (Sigma) and ii. Hetasep (Stem Cell Technologies).

PBMCs were resuspended in 200 µl of Actiphage^{™} media and samples were then transferred to Actiphage^{™} Rapid Tubes (PBD Biotech Ltd) and bacteriophage D29 was added (20 µl; ~10⁷ pfu). The samples were incubated for 3.5 hrs at 37 °C and then centrifuged (13,000 xg; 3 mins, room temperature). Flow-through from the Rapid tubes, containing the released mycobacterial DNA, was further concentrated (Zymo DNA Clean and Concentrator-5; Zymo Research / Cambridge Bioscience) and Mtb DNA was detected using a PCR assay specific for the IS6110 element using forward and reverse primers 5'-CCTGCGAGCGTAGGCGTCGG-3' and 5'-CTCGTCCAGCGCCGCTTCGG-3' that generate a 123 bp PCR product (Eisenach et al., Detection of Mycobacterium tuberculosis in sputum samples using a polymerase chain reaction. Am Rev Respir Dis. 1991 Nov;144(5):1160-3).

Sixty-six participants were recruited to the study (Table 1). Of the 15 participants with active PTB (group 1), one had evidence of miliary disease, with a single cerebral tuberculoma. For the remainder of group 1, there were no radiological or clinical features of multi-organ involvement. Of the 18 group 2 participants, one had QFT seroconversion with serial testing. The remainder were QFT positive at both time-points and all had normal chest X-rays reported by a thoracic radiologist. All five participants of the control group with non-TB acute respiratory illness (group 3) had PTB excluded with bronchoscopy and were treated effectively with antibiotics for community acquired pneumonia.

Table 1 shows that 11 out of the 15 (73%) active PTB cohort (group 1) , and 3 out of the 18 asymptomatic TB-contact participants (group 2) gave a positive Actiphage^{™} bacteriophage-mediated Mycobacterial DNA release assay test result (these were participants assigned participant numbers 455, 493 and 494). The remaining 4 group 1 participants, 15 group 2 participants, and all participants of both control groups (group 3 and group 4) gave a negative Actiphage^{™} bacteriophage-mediated Mycobacterial DNA release assay test result.

Figure 3 shows positive PCR bands of the expected size (123 bp) for one of the group 2 participants (assigned participant number 455) when using primers specific for the Mycobacterial IS6110 element.

All study participants were also tested for C-reactive protein (CRP) levels to determine the presence of inflammation. In addition, all participants in group 1, group 3, and the Actiphage-positive participants in group 2 were also tested by sputum smear microscopy, by Xpert-Ultra test, and by measuring days to positive mycobacterial culture from respiratory tract samples.

In the PTB cohort (group 1, active TB disease), a positive Actiphage^{™} bacteriophage-mediated Mycobacterial DNA release assay result was associated with sputum smear positivity, higher baseline CRP and shorter time to mycobacterial culture. In the asymptomatic TB-contact group (group 2), participants had normal (in a similar range to healthy controls) baseline CRP and the lack of ability to form mycobacterial culture at baseline (i.e. no detectable bacterial growth after 8 weeks incubation). Of the 3 Actiphage^{™}-positive participants identified at baseline in group 2, two developed active, culture-positive PTB disease after 7 months. The lack of clinical, radiological and microbiological evidence of TB at presentation, combined with development of active disease at a later timepoint, is consistent with a diagnosis of incipient TB. This demonstrates the utility of the bacteriophage-mediated Mycobacterial DNA release assay in diagnosis of incipient TB, and its ability to distinguish subjects with incipient TB from those with LTBI. Whole genome sequence analysis of the Mtb isolates from the two group 2 cases who went on to develop active TB confirmed bacterial origin from their respective index cases. The third Actiphage^{™}-positive group 2 subject had QFT seroconversion (i.e. gave a negative QFT result on first testing followed by a positive result 3 months later) but did not develop active TB within the timescale of the study (12 months). After 12-months follow up, no Actiphage^{™}-negative participants from group 2 had developed active TB. Therefore the bacteriophage-mediated Mycobacterial DNA release assay appeared not to generate any false-negative active TB results amongst the group 2 cohort, demonstrating that it is an effective test for incipient TB. Additionally, those asymptomatic cases who tested negative using the bacteriophage-mediated Mycobacterial DNA release assay (Actiphage^{™}), and were positive by QFT testing, could be considered to have latent TB infection (i.e. low risk of developing active TB disease) with greater confidence than by QFT testing alone.

As a clinical diagnostic in symptomatic patients with suspected active PTB at baseline (i.e. groups 1 and 3), the bacteriophage-mediated Mycobacterial DNA release assay (Actiphage^{™} Rapid test) had a sensitivity and specificity (95 % Cl) of 73.3 % (48.1-89.1) and 100 % (56.6-100), respectively.

As a clinical diagnostic for incipient TB (high risk of developing active TB) in asymptomatic patients at baseline (i.e. groups 2 and 4), the bacteriophage-mediated Mycobacterial DNA release assay (Actiphage^{™} Rapid test) had a sensitivity and specificity (95 % Cl) of 100.0 % (15.8-100.0) and 97.3 % (88.0-99.9), respectively.

When applied to the whole cohort at baseline (all groups), sensitivity and specificity (95% Cl) for detecting active PTB were 73.3 % (48.1-89.1) and 94.2% (84.1-98.4), respectively.

**Table 1. Demographic and clinical characteristics of all subjects in this study. (BCG, Bacillus Calmette-Guérin; TB, Tuberculosis; BMI, Body Mass Index; CRP, C-Reactive Protein; S. aureus, Staphylococcus aureus; M. avium, Mycobacterium avium). * Data presented are at the time of presentation (i.e. at 7 months after baseline) with active (culture positive) TB in two contacts, ^ CRP values refer to data collected at baseline, consistent with the data for the other groups, ^{$} Percentages calculated from the subgroup for which BCG status was known.**

| | | **Symptomatic patients** | | |
|---|---|---|---|---|
| | | **Active TB disease (PTB) (N=15)** | | **Non-TB Acute Respiratory Illness (N=5)** |
| **Actiphage^{™} Result at baseline** | | Positive (n=11) | Negative (n=4) | Negative |
| **Male Gender** (%) | | 5 (45.5) | 2(50) | 2(40) |
| **Age** (years; mean ±SD) | | 31.5 (±13.9) | 38.8 (±13.5) | 50 (±21.7) |
| **UK Born** (%) | | 3 (27.2) | 1 (25) | 2(40) |
| **BCG Vaccination** | Yes (%)^{$} | 4 (36.4) | 2 (50) | 2(40) |
| | Unknown (%) | 0 | 0 | 0 |
| **BMI** (kg/m²; mean ±SD) | | 19.9 (±3.6) | 20.9 (±3.0) | 25.7 (±5.3) |
| **TB Disease Characteristics** | Smear Positive | 7 | 0 | 0 |
| | Smear Negative | 4 | 4 | 0 |
| | Xpert-Ultra Grade | Medium - High | Very Low - Low | All Negative |
| | CRP (median, IQR) | 63 (36 to 65) | 41 (27 to 45.5) | 84 (45 to 110) |
| | Days to Positive Mycobacterial Culture (median, IQR) | 15 (10.5 to 22) | 21 (21 to 21) | 1 blood culture (*S. aureus*) |
| | | | | 1 sputum culture (*M. avium,* 6 days) |

| | | **Asymptomatic patients** | | |
|---|---|---|---|---|
| | | **Recent Pulmonary TB Contacts with positive QFT(N=18)** | | **Healthy Controls: No positive QFT or TB contact (N=28)** |
| **Actiphage^{™} Result at baseline** | | Positive (n=3) | Negative (n=15) | Negative |
| **Male Gender** (%) | | 1 (33.3) | 10 (55.6) | 11 (39.3) |
| **Age** (years; mean ±SD) | | 25.3 (±6.4) | 54.7 (±12.3) | 38.9 (±14.6) |
| **UK Born** (%) | | 1 (33.3) | 5 (33.3) | 10 (35.7) |
| **BCG Vaccination** | Yes (%)^{$} | 2 (66.7) | 7 (63.6) | 12 (50) |
| | Unknown (%) | 0 | 4 (26.7) | 4 (14.3) |
| **BMI** (kg/m²; mean ±SD) | | 21.9 (±2.0) | 26.2 (±6.9) | 27.1 (±8.2) |
| **TB Disease Characteristics** | Smear Positive | 0 | N/A | N/A |
| | Smear Negative | 2 | N/A | N/A |
| | Xpert-Ultra Grade | Medium* | N/A | N/A |
| | CRP (median, IQR) | 5 (5 to 5)^ | 10 (5 to 13.75) | 5 (5 to 10) |
| | Days to Positive Culture (median, IQR) | 26 (23.5 to 28.5)* | N/A | N/A |

## Claims

1. A method of diagnosis of a disease in an asymptomatic human subject, the method comprising:
obtaining a sample of peripheral blood mononuclear cells (PBMCs) isolated from the subject;
admixing Mycobacteria-specific bacteriophage D29 with the sample of PBMCs to produce an admixture;
incubating the admixture under conditions that permit lysis of Mycobacteria;
isolating DNA from the admixture;
determining if a Mycobacterial DNA sequence is present or absent in the DNA isolated from the admixture by one or more of: performing PCR on the DNA isolated from the admixture using forward and reverse primers specific for the Mycobacterial DNA sequence, and/or performing sequencing suitable to identify the Mycobacterial DNA sequence, and optionally determining homology between the Mycobacterial DNA sequence, if present, and known Mycobacterial DNA sequences;
performing a Mycobacterium-specific cell-mediated immune (CMI) response test on a sample isolated from the subject to generate a positive or negative CMI response test outcome;
wherein the asymptomatic human subject is diagnosed as having incipient tuberculosis (TB) when the Mycobacterial DNA sequence is present, with either a positive or negative CMI response test outcome, where present; and
wherein the asymptomatic human subject is diagnosed as having latent tuberculosis infection (LTBI) when the Mycobacterial DNA sequence is absent and a positive CMI response test outcome is present.

2. The method according to claim 1, wherein the Mycobacterial DNA sequence comprises the IS6110 element or the IS900 element.

3. The method according to claim 1 or claim 2, wherein the asymptomatic human subject is a TB-contact subject.

4. The method according to claim 3, wherein the method is performed on a sample of PBMCs obtained from the asymptomatic human subject within 12 months, 6 months, 5 months, 4 months, 3 months, 2 months or 1 month of the asymptomatic human subject coming into contact with a TB-infected individual.

5. The method according to any preceding claim, wherein incubating the admixture under conditions that permit lysis of Mycobacteria comprises incubation at approximately 37 °C for approximately or less than 6 hours, 5 hours, 4 hours, 3.5 hours, 3 hours, 2.5 hours, 2 hours, 1.5 hours or 1 hour, preferably for approximately 3.5 hours.

6. The method according to any preceding claim, wherein the optional CMI response test comprises an interferon-gamma release assay (IGRA) test.

7. A method of predicting if a subject is at risk of developing or having incipient tuberculosis (TB), the method comprising:
obtaining a sample of peripheral blood mononuclear cells (PBMCs) isolated from the subject;
admixing a Mycobacteria-specific bacteriophage with the sample of PBMCs to produce an admixture;
incubating the admixture under conditions that permit lysis of Mycobacteria;
isolating DNA from the admixture;
identifying if Mycobacterial DNA sequences are present in the DNA;
wherein the presence of Mycobacterial DNA sequences indicates that the subject is at risk of developing or having incipient TB.

8. A method of assessing the tuberculosis (TB) disease prognosis for a subject, the method comprising:
obtaining a sample of peripheral blood mononuclear cells (PBMCs) isolated from the subject;
admixing a Mycobacteria-specific bacteriophage with the sample of PBMCs to produce an admixture;
incubating the admixture under conditions that permit lysis of Mycobacteria;
isolating DNA from the admixture;
identifying if Mycobacterial DNA sequences are present in the DNA isolated from the admixture;
wherein the presence of Mycobacterial DNA sequences indicates that the subject is at risk of having incipient TB and/or developing active TB disease.

9. A composition comprising one or more anti-tuberculosis medicament for use in a method of treating tuberculosis (TB) in a subject in need thereof, the method comprising:
obtaining a sample of peripheral blood mononuclear cells (PBMCs) isolated from the subject;
using a bacteriophage-mediated Mycobacterial DNA release assay to identify the presence of incipient TB in the subject; and, wherein the composition is administered to the subject where incipient TB is identified.

10. A composition comprising one or more anti-incipient tuberculosis medicament for use in a method of treating incipient tuberculosis (TB) in a subject in need thereof, the method comprising:
obtaining a sample of peripheral blood mononuclear cells (PBMCs) isolated from the subject;
admixing a Mycobacteria-specific bacteriophage with the sample of PBMCs to produce an admixture;
incubating the admixture under conditions that permit lysis of Mycobacteria;
isolating DNA from the admixture;
identifying the presence of Mycobacterial sequences in the DNA; and
administering the compound to the subject where Mycobacterial sequences are identifed in the DNA.

11. A composition comprising one or more anti-tuberculosis medicament for use in a method of treating tuberculosis comprising improving tuberculosis (TB) prognosis in a subject, the method comprising:
obtaining a sample of peripheral blood mononuclear cells (PBMCs) isolated from the subject;
using a bacteriophage-mediated Mycobacterial DNA release assay to identify the presence of incipient TB in the subject; and, wherein the composition is administered to the subject where incipient TB is identified.

12. Use of a bacteriophage for measuring in a blood sample isolated from a human subject the presence of one or more Mycobacterial biomarker for assessing if said human subject is at risk of developing or has incipient tuberculosis (TB) or active tuberculosis disease, optionally, in the form of a kit.

## Patentansprüche

1. Verfahren zur Diagnose einer Erkrankung bei einem asymptomatischen menschlichen Subjekt, wobei das Verfahren Folgendes umfasst:
Erhalten einer Probe von peripheren mononukleären Blutzellen (PBMC), die aus dem Subjekt isoliert sind;
Mischen des Mykobakterien-spezifischen Bakteriophagen D29 mit der PBMC-Probe, um eine Mischung herzustellen;
Inkubieren der Mischung unter Bedingungen, welche die Lyse von Mykobakterien ermöglichen;
Isolieren von DNA aus der Mischung;
Bestimmen, ob eine mykobakterielle DNA-Sequenz in der aus der Mischung isolierten DNA vorhanden ist oder nicht vorhanden ist, durch eines oder mehrere von Folgendem: Durchführen einer PCR an der aus der Mischung isolierten DNA unter Verwendung von Vorwärts- und Rückwärtsprimern, die für die mykobakterielle DNA-Sequenz spezifisch sind, und/oder Durchführen einer Sequenzierung, die zum Identifizieren der mykobakteriellen DNA-Sequenz geeignet ist, und optional Bestimmen einer Homologie zwischen der mykobakteriellen DNA-Sequenz, falls vorhanden, und bekannten mykobakteriellen DNA-Sequenzen;
Durchführen eines Mycobacterium-spezifischen zellvermittelten Immunreaktionstests (CMI-Tests) an einer aus dem Subjekt isolierten Probe, um ein positives oder negatives CMI-Reaktionstestergebnis zu generieren; wobei bei dem asymptomatischen menschlichen Subjekt diagnostiziert wird, dass es eine inzipiente Tuberkulose (TB) aufweist, wenn die mykobakterielle DNA-Sequenz vorhanden ist, mit entweder einem positiven oder negativen CMI-Reaktionstestergebnis, sofern vorhanden, und
wobei bei dem asymptomatischen menschlichen Subjekt diagnostiziert wird, dass es eine latente Tuberkulose-Infektion (LTBI) aufweist, wenn die mykobakterielle DNA-Sequenz nicht vorhanden ist und ein positives CMI-Reaktionstestergebnis vorhanden ist.

2. Verfahren nach Anspruch 1, wobei die mykobakterielle DNA-Sequenz das IS6110-Element oder das IS900-Element umfasst.

3. Verfahren nach Anspruch 1 oder Anspruch 2, wobei es sich bei dem asymptomatischen menschlichen Subjekt um ein Subjekt mit TB-Kontakt handelt.

4. Verfahren nach Anspruch 3, wobei das Verfahren an einer PBMC-Probe durchgeführt wird, die von dem asymptomatischen menschlichen Subjekt innerhalb von 12 Monaten, 6 Monaten, 5 Monaten, 4 Monaten, 3 Monaten, 2 Monaten oder 1 Monat erhalten wird, nachdem das asymptomatische menschliche Subjekt in Kontakt mit einer TB-infizierten Person gekommen ist.

5. Verfahren nach einem der vorhergehenden Ansprüche, wobei das Inkubieren der Mischung unter Bedingungen, die eine Lyse von Mykobakterien ermöglichen, eine Inkubation bei etwa 37 °C für etwa oder weniger als 6 Stunden, 5 Stunden, 4 Stunden, 3,5 Stunden, 3 Stunden, 2,5 Stunden, 2 Stunden, 1,5 Stunden oder 1 Stunde, vorzugsweise etwa 3,5 Stunden, umfasst.

6. Verfahren nach einem der vorhergehenden Ansprüche, wobei der optionale CMI-Reaktionstest einen Interferon-Gamma-Release-Assay-(IGRA-)Test umfasst.

7. Verfahren zum Prognostizieren, ob bei einem Subjekt das Risiko besteht, eine inzipiente Tuberkulose (TB) zu entwickeln oder aufzuweisen, wobei das Verfahren Folgendes umfasst:
Erhalten einer Probe von peripheren mononukleären Blutzellen (PBMC), die aus dem Subjekt isoliert sind;
Mischen eines Mykobakterien-spezifischen Bakteriophagen mit der PBMC-Probe, um eine Mischung herzustellen;
Inkubieren der Mischung unter Bedingungen, welche die Lyse von Mykobakterien ermöglichen;
Isolieren von DNA aus der Mischung;
Identifizieren, ob mykobakterielle DNA-Sequenzen in der DNA vorhanden sind;
wobei das Vorhandensein von mykobakteriellen DNA-Sequenzen angibt, dass bei dem Subjekt das Risiko besteht, eine inzipiente TB zu entwickeln oder aufzuweisen.

8. Verfahren zum Beurteilen der Tuberkulose-(TB-)Erkrankungsprognose für ein Subjekt, wobei das Verfahren Folgendes umfasst:
Erhalten einer Probe von peripheren mononukleären Blutzellen (PBMC), die aus dem Subjekt isoliert sind;
Mischen eines Mykobakterien-spezifischen Bakteriophagen mit der PBMC-Probe, um eine Mischung herzustellen;
Inkubieren der Mischung unter Bedingungen, welche die Lyse von Mykobakterien ermöglichen;
Isolieren von DNA aus der Mischung;
Identifizieren, ob mykobakterielle DNA-Sequenzen in der aus der Mischung isolierten DNA vorhanden sind;
wobei das Vorhandensein von mykobakteriellen DNA-Sequenzen angibt, dass bei dem Subjekt das Risiko besteht, eine inzipiente TB aufzuweisen und/oder eine aktive TB-Erkrankung zu entwickeln.

9. Zusammensetzung, umfassend ein oder mehrere Anti-Tuberkulose-Medikamente zur Verwendung bei einem Verfahren zum Behandeln von Tuberkulose (TB) bei einem Subjekt, das dessen bedarf, wobei das Verfahren Folgendes umfasst:
Erhalten einer Probe von peripheren mononukleären Blutzellen (PBMC), die aus dem Subjekt isoliert sind;
Verwenden eines Bakteriophagen-vermittelten mykobakteriellen DNA-Release-Assays, um das Vorhandensein einer inzipienten TB bei dem Subjekt zu identifizieren; und wobei die Zusammensetzung dem Subjekt verabreicht wird, bei dem eine inzipiente TB identifiziert wird.

10. Zusammensetzung, umfassend ein oder mehrere Medikamente gegen inzipiente Tuberkulose zur Verwendung bei einem Verfahren zum Behandeln inzipienter Tuberkulose (TB) bei einem Subjekt, das dessen bedarf, wobei das Verfahren Folgendes umfasst:
Erhalten einer Probe von peripheren mononukleären Blutzellen (PBMC), die aus dem Subjekt isoliert sind;
Mischen eines Mykobakterien-spezifischen Bakteriophagen mit der PBMC-Probe, um eine Mischung herzustellen;
Inkubieren der Mischung unter Bedingungen, welche die Lyse von Mykobakterien ermöglichen;
Isolieren von DNA aus der Mischung;
Identifizieren des Vorhandenseins von mykobakteriellen Sequenzen in der DNA; und
Verabreichen der Verbindung an das Subjekt, bei dem mykobakterielle Sequenzen in der DNA identifiziert werden.

11. Zusammensetzung, umfassend ein oder mehrere Anti-Tuberkulose-Medikamente zur Verwendung bei einem Verfahren zum Behandeln von Tuberkulose, das Verbessern einer Tuberkulose-(TB-)Prognose bei einem Subjekt umfasst, wobei das Verfahren Folgendes umfasst:
Erhalten einer Probe von peripheren mononukleären Blutzellen (PBMC), die aus dem Subjekt isoliert sind;
Verwenden eines Bakteriophagen-vermittelten mykobakteriellen DNA-Release-Assays, um das Vorhandensein einer inzipienten TB bei dem Subjekt zu identifizieren; und wobei die Zusammensetzung dem Subjekt verabreicht wird, bei dem eine inzipiente TB identifiziert wird.

12. Verwenden eines Bakteriophagen zum Messen des Vorhandenseins eines oder mehrerer mykobakterieller Biomarker in einer von einem menschlichen Subjekt isolierten Blutprobe, um zu beurteilen, ob bei dem menschlichen Subjekt das Risiko besteht, inzipiente Tuberkulose (TB) oder eine aktive Tuberkulose-Erkrankung zu entwickeln, oder ob es diese aufweist, optional in Form eines Kits.

## Revendications

1. Méthode de diagnostic d'une maladie chez un sujet humain asymptomatique, la méthode comprenant :
l'obtention d'un échantillon de cellules mononucléées de sang périphérique (PBMC) isolées du sujet ;
le mélange du bactériophage D29 spécifique aux mycobactéries avec l'échantillon de PBMC pour produire un mélange ;
l'incubation du mélange dans des conditions qui permettent la lyse des mycobactéries ;
l'isolement de l'ADN du mélange ;
la détermination pour savoir si une séquence d'ADN mycobactérienne est présente ou absente dans l'ADN isolé du mélange par une ou plusieurs des opérations suivantes : la réalisation d'une PCR sur l'ADN isolé du mélange en utilisant des amorces directes et inverses spécifiques à la séquence d'ADN mycobactérienne, et/ou la réalisation d'un séquençage approprié pour identifier la séquence d'ADN mycobactérienne, et éventuellement la détermination de l'homologie entre la séquence d'ADN mycobactérienne, si elle est présente, et les séquences d'ADN mycobactériennes connues ;
la réalisation d'un test de réponse immunitaire à médiation cellulaire (CMI) spécifique aux mycobactéries sur un échantillon isolé du sujet pour générer un résultat de test de réponse CMI positif ou négatif ;
ledit sujet humain asymptomatique étant diagnostiqué comme étant atteint de tuberculose (TB) naissante lorsque la séquence d'ADN mycobactérienne est présente, avec un résultat de test de réponse CMI positif ou négatif, le cas échéant ; et
ledit sujet humain asymptomatique étant diagnostiqué comme étant atteint d'une infection tuberculeuse latente (LTBI) lorsque la séquence d'ADN mycobactérienne est absente et qu'un résultat de test de réponse CMI positif est présent.

2. Méthode selon la revendication 1, ladite séquence d'ADN mycobactérienne comprenant l'élément IS6110 ou l'élément IS900.

3. Méthode selon la revendication 1 ou la revendication 2, ledit suj et humain asymptomatique étant un sujet ayant été en contact avec la TB.

4. Méthode selon la revendication 3, ladite méthode étant effectuée sur un échantillon de PBMC obtenu à partir du sujet humain asymptomatique dans un délai de 12 mois, 6 mois, 5 mois, 4 mois, 3 mois, 2 mois ou 1 mois suivant la venue en contact du sujet humain asymptomatique avec une personne infectée par la TB.

5. Méthode selon l'une quelconque des revendications précédentes, ladite incubation du mélange dans des conditions qui permettent la lyse des mycobactéries comprenant l'incubation à environ 37 °C pendant environ ou moins de 6 heures, 5 heures, 4 heures, 3,5 heures, 3 heures, 2,5 heures, 2 heures, 1,5 heures ou 1 heure, de préférence pendant environ 3,5 heures.

6. Méthode selon l'une quelconque des revendications précédentes, ledit test facultatif de réponse CMI comprenant un test de libération d'interféron gamma (IGRA).

7. Méthode de prédire si un sujet présente un risque de développer ou d'être ateint de tuberculose (TB) naissante, la méthode comprenant :
l'obtention d'un échantillon de cellules mononucléées de sang périphérique (PBMC) isolées du sujet ;
le mélange d'un bactériophage spécifique aux mycobactéries avec l'échantillon de PBMC pour produire un mélange ;
l'incubation du mélange dans des conditions qui permettent la lyse des mycobactéries ;
l'isolement de l'ADN du mélange ;
l'identification pour déterminer si des séquences d'ADN mycobactériennes sont présentes dans l'ADN ;
ladite présence de séquences d'ADN mycobactériennes indiquant que le sujet présente un risque de développer une TB naissante ou d'en être atteint.

8. Méthode d'évaluer un pronostic de tuberculose (TB) chez un sujet, la méthode comprenant :
l'obtention d'un échantillon de cellules mononucléées de sang périphérique (PBMC) isolées du sujet ;
le mélange d'un bactériophage spécifique aux mycobactéries avec l'échantillon de PBMC pour produire un mélange ;
l'incubation du mélange dans des conditions qui permettent la lyse des mycobactéries ;
l'isolement de l'ADN du mélange ;
l'identification pour déterminer si des séquences d'ADN mycobactériennes sont présentes dans l'ADN isolé du mélange ; ladite présence de séquences d'ADN mycobactériennes indiquant que le sujet présente un risque d'avoir une TB naissante et/ou de développer une TB active.

9. Composition comprenant un ou plusieurs médicaments anti-tuberculose destinée à être utilisée dans une méthode de traitement de la tuberculose (TB) chez un sujet en ayant besoin, la méthode comprenant :
l'obtention d'un échantillon de cellules mononucléées de sang périphérique (PBMC) isolées du sujet ;
l'utilisation d'un test de libération d'ADN mycobactérien médié par bactériophage pour identifier la présence d'une TB naissante chez le sujet ; et ladite composition étant administrée au sujet chez lequel une TB naissante est identifiée.

10. Composition comprenant un ou plusieurs médicaments anti-tuberculose naissante destinée à être utilisée dans une méthode de traitement de la tuberculose (TB) naissante chez un sujet en ayant besoin, la méthode comprenant :
l'obtention d'un échantillon de cellules mononucléées de sang périphérique (PBMC) isolées du sujet ;
le mélange d'un bactériophage spécifique aux mycobactéries avec l'échantillon de PBMC pour produire un mélange ;
l'incubation du mélange dans des conditions qui permettent la lyse des mycobactéries ;
l'isolement de l'ADN du mélange ;
l'identification de la présence de séquences mycobactériennes dans l'ADN ; et
l'administration du composé au sujet chez lequel des séquences mycobactériennes sont identifiées dans l'ADN.

11. Composition comprenant un ou plusieurs médicaments anti-tuberculose destinée à être utilisée dans une méthode de traitement de la tuberculose comprenant
l'amélioration du pronostic de tuberculose (TB) chez un sujet, la méthode comprenant :
l'obtention d'un échantillon de cellules mononucléées de sang périphérique (PBMC) isolées du sujet ;
l'utilisation d'un test de libération d'ADN mycobactérien médié par bactériophage pour identifier la présence d'une TB naissante chez le sujet ; et ladite composition étant administrée au sujet chez lequel une TB naissante est identifiée.

12. Utilisation d'un bactériophage pour mesurer dans un échantillon de sang isolé d'un sujet humain la présence d'un ou plusieurs biomarqueurs mycobactériens pour évaluer si ledit sujet humain présente un risque de développer une tuberculose (TB) naissante ou une tuberculose active ou en est atteint, éventuellement, sous la forme d'un kit.
